# EUROPÄISCHE PATENTSCHRIFT

(11) **EP 3 148 371 B1**
(45) Veröffentlichungstag und Bekanntmachung des Hinweises auf die Patenterteilung: **04.09.2019**
(21) Anmeldenummer: 15725013.5
(22) Anmeldetag: 27.05.2015
(51) Int. Cl.: A46B 15/00, A46D 1/00, A61C 3/00, A61K 6/00

(54) **REAKTIVER MIKRO-APPLIKATOR MIT METALL ENTHALTENDEN ADDITIVEN ZUR VERWENDUNG MIT DENTAL ADHÄSIVEN**
REACTIVE MICRO APPLICATOR WITH METAL-CONTAINING ADDITIVES FOR USE IN DENTAL ADHESIVES
MICRO-APPLICATEUR RÉACTIF AVEC ADDITIFS À TENEUR EN MÉTAL POUR UTILISATION AVEC DES ADHÉSIFS DENTAIRES

(30) Priorität: 28.05.2014 DE 102014107518
(43) Veröffentlichungstag der Anmeldung: 05.04.2017
(73) Patentinhaber: Kulzer GmbH, 63450 Hanau (DE)
(72) Erfinder: LOH, (GEB. MÜLLER), Waltraut, 60318 Frankfurt (DE); KASTRATI, Astrit, 61206 Wöllstadt (DE); QUINQUE, Bärbel, 61169 Friedberg-Ockstadt (DE); EPPINGER, Regina, 35781 Weilburg (DE); HOFFMANN, Marcus, 61250 Usingen (DE)
(74) Vertreter: Bendele, Tanja
(86) Internationale Anmeldenummer: PCT/EP2015/061692
(87) Internationale Veröffentlichungsnummer: WO 2015/181227

(56) Entgegenhaltungen:
- KR-B1- 100 854 960
- US-A- 5 407 973
- US-A1- 2008 286 713
- US-A1- 2009 005 469
- US-A1- 2013 143 176

## Beschreibung

Die Erfindung betrifft einen dentalen Mikro-Applikator mit einem Körper, wobei der Körper ein distales und ein proximales Ende aufweist, und an dem proximalen Ende ein Applikationsmittel zumindest teilweise mit mindestens einem Additiv versehen ist, ausgewählt aus Metallen, Metall enthaltenden Verbindungen und metallorganischen Verbindungen zur Beeinflussung der Polymerisation dentaler Materialien, sowie ein Verfahren zu seiner Herstellung als auch ein Kit umfassend den Applikator und ein dentales Adhäsiv. Dokument US 2013/143176 A1, z.B. offenbart einen dentalen Mikro-Applikator mit einem Applikationsmittel, wobei mit dem Applikationsmittel ein Adhäsiv mit nanopartikulärem Metalloxid appliziert wird.

Zur Verbesserung der Haftung dentaler Materialien an Zähnen sind vielfältige Bondings und Primer Kombinationen bekannt. Die Haftmittel der jüngeren Generation umfassen einkomponentige, selbstätzende Haftmittel. Um eine gute Haftung auf dem Zahn zu gewährleisten muss eine große, gut benetzbare Haftfläche bereitgestellt werden. Das Adhäsiv selbst muss dünnflüssig sein, gute benetzende Eigenschaften aufweisen und sehr schnell aushärten, um die Möglichkeit eines Kontaktes mit Feuchtigkeit mit der Folge einer verminderten Haftung zu minimieren.

So sind selbstätzende Adhäsive bekannt, die die Schritte der Säureätzung und des anschließenden Applizierens des Adhäsivs in einem Schritt zusammenfassen. Das säurehaltige Adhäsivsystem löst die Schmierschicht auf und legt das darunterliegende Dentin frei oder löst die Schmierschicht lediglich an, um diese permeabel für Inhaltsstoffe des Adhäsivs zu machen. Simultan erfolgt eine Infiltration der Monomere in die Zahnhartsubstanz. Im Falle des Schmelzes wird durch die säurehaltigen Adhäsivsysteme ein der Phosphorsäureätzung ähnliches Ätzmuster erzeugt. Das zur Infiltration notwendige Lösungsmittel wird mit einem Luftpüster entfernt und das Adhäsiv wird gehärtet, bspw. strahlengehärtet.

Aufgabe der Erfindung war es die Härtung polymerisierbarer dentaler Materialien zu verbessern bzw. zu beschleunigen. Ebenso sollte die Scherbindungsfestigkeit der dentalen Materialien weiter verbessert werden. Ferner bestand die Aufgabe ein Mittel und ein Kit bereitzustellen mit dem die Polymerisation und vorzugsweise darüber die Härtung und vorzugsweise die Haftung dentaler Materialien am Dentin und/oder am Schmelz verbessert werden kann. Eine weitere Aufgabe bestand darin, dass vorzugsweise keine weiteren Arbeitsschritte beim Anwender notwendig werden, um die Polymerisation und vorzugsweise die Härtung zu beschleunigen und vorzugsweise die Haftung am Zahn zu verbessern. Des Weiteren sollte die Behandlungszeit vorteilhaft verkürzt werden.

Gelöst werden die Aufgaben mit einem dentalen Mikro-Applikator nach Anspruch 1, dem Verfahren nach Anspruch 9 zur Herstellung des Applikator sowie dem Kit nach Anspruch 13 umfassend den Applikator als auch durch die erfindungsgemäße Verwendung nach Anspruch 16. Bevorzugte Ausführungsformen sind in den Unteransprüchen beschrieben sowie detailliert in der Beschreibung.

Gelöst werden die Aufgaben der Erfindung mit einem Mikro-Applikator, der mit mindestens einem Additiv versehen ist, das vorzugsweise katalytisch aktives Platin umfasst, vorzugsweise umfasst der Applikator Platinschwamm sowie optional mindestens ein weiteres Additiv sowie optional mindestens einen Hilfsstoff. Als weiteres Additiv haben sich basische Additive bewährt, die in der Lage sind retardiert den pH-Wert der selbstätzenden Haftmittel, synonym selbstätzenden Adhäsiven nach dem Ätzschritt zu erhöhen. Als Hilfsstoffe können beispielsweise Sprengmittel (Zerfallsmittel) oder Filmbildner wie Zellulose, mikrokristalline Zellulose eingesetzt werden.

Gegenstand der Erfindung ist ein dentaler Mikro-Applikator mit einem Körper, insbesondere mit einem länglichen stabförmigen Körper, mit einem distalen und einem proximalen Ende, wobei an dem proximalen Ende ein Applikationsmittel ist, wobei das Applikationsmittel mindestens an seiner äußeren Oberfläche, insbesondere optional an der inneren Oberfläche, zumindest teilweise mindestens ein Additiv aufweist, ausgewählt aus Metallen, Metall enthaltenden Verbindungen und metallorganischen Verbindungen zur Beeinflussung der Polymerisation nach Anspruch 1, insbesondere zur Beschleunigung der Polymerisation und/oder zur Verbesserung der chemischen Beständigkeit des polymerisierten dentalen Materials. Dabei ist es besonders bevorzugt, wenn eine radikalische Polymerisation beeinflusst, vorzugsweise beschleunigt wird. Erfindungsgemäß wird die Polymerisation von dentalen Adhäsiven, wie selbstätzenden Adhäsiven, insbesondere von dentalen Monomeren mit mindestens einer Säuregruppe, vorzugsweise von dentalen Adhäsiven mit einem pH-Wert unter pH = 7, vorzugsweise mit einem pH-Wert von 6 bis 1, bevorzugt von 5 bis 1, beschleunigt. Die radikalisch polymerisierbaren Monomere mit einer Säuregruppe im Molekül, auch saure Komponente genannt, umfassen polymerisierbare Monomere mit mindestens einer, vorzugsweise mehreren Ethylen-Gruppen sowie mindestens eine Carbonsäure-, Carbonsäureanhydrid-, Phosphorsäure- und/oder Sulfonsäure-Gruppe, wie sie nachfolgend näher offenbart sind.

Vorzugsweise wird die Haftung dentaler Materialien verbessert und/oder die chemische Kompatibilität mit weiteren dentalen Materialien verbessert. Bevorzugt kann der Applikator auch eine Mischung der vorgenannten Additive aufweisen. Vorteilhaft weist der Applikator 1 bis 10 verschiedene Additive, Mischungen dieser Additive, Aktivatoren und/oder Hilfsmittel auf, insbesondere 1, 2, 3, 4, 5, 6, 7, 8, 9 oder 10.

Mit einem besonders bevorzugten Mikro-Applikator, der mit Platin, vorzugsweise pulverförmigem Platin, versehen ist, konnte eine Verbesserung der Haftwerte nachgewiesen werden. Zur Herstellung des Applikators wurde ein handelsüblicher Applikator mit einer wässrigen Nano-Platin Suspension behandelt und getrocknet. Mit diesem so behandelten Applikator wurde ein positiver Effekt bei einer Vorbehandlung der Zahnoberfläche in Kombination mit Heraeus iBOND® Self Etch nachgewiesen. Im Vergleich zu den Referenzversuchen mit iBOND® Self Etch mit einem handelsüblichen Applikator (ohne Platin behandelt) wurden bei Verwendung eines mit Platin behandelten Applikators statistisch signifikant höhere Scherhaftungsergebnisse am Zahnschmelz erhalten. Die Scherhaftungsergebnisse auf Schmelz konnten mit diesen Applikatoren um 10 bis 20% gesteigert werden. Die insgesamt erzielte Haftung konnte somit weiter in einen bislang nicht erzielbaren Bereich verbessert werden.

Besonders bevorzugt ist der Applikator oder das Applikationsmittel, insbesondere an seiner äußeren Oberfläche und/oder an mindestens einer inneren Oberfläche, zumindest teilweise mit nanopartikulärem Platin imprägniert. Weiter bevorzugt weist das nanopartikuläre Platin eine Partikelgröße kleiner gleich 10 nm auf, insbesondere ist das nanopartikuläre Platin Platinschwamm. Besonders bevorzugte Partikelgrößen des Platins umfassen Partikel kleiner gleich 50 nm, kleiner gleich 10 nm, 7 nm, um 5 nm mit +/- 2 nm. Besonders bevorzugt ist ein Platin-Schwamm. Die Reinheit des Platins beträgt vorzugsweise größer gleich 90 Gew.-% eines Gehaltes an Platin, insbesondere größer gleich 95 Gew.-%, 98 Gew.-%, 99 Gew.-%, 99,5 %-Gew, weiter bevorzugt größer gleich 99,7 Gew.-%, 99,8 Gew.-%, 99,9 Gew.-%.

Nach einer bevorzugten Alternative werden Applikatoren bereitgestellt, die zusätzlich zu einem ersten Additiv mindestens ein weiteres Additiv aufweisen, insbesondere einen Aktivator. Dabei ist eine Kombination von Platin und einem Salz einer Sulfinsäure, insbesondere ein Alkalisalz der Toluolsulfinsäure, eine bevorzugte Kombination von Additiven auf den Applikatoren. Mit dieser Kombination ist es möglich nicht lagerfähige oder nicht mit einer dentalen Zusammensetzung kompatible Verbindung weiterhin in einer Ein-Schritt-Anwendung zu applizieren.

So wurden Applikatoren mit Platin imprägniert. Die so erhaltenen getrockneten Applikatoren wurden verwendet, um iBOND® Self Etch auf Zähnen, insbesondere in den Kavitäten der Zähne, besonders bevorzugt im Zusammenhang mit einer Karriesbehandlung, aufzutragen. Als Vergleich dienen die Proben von iBOND®, die mit unbehandelten Applikatoren aufgetragen wurden. Die Scherhaftfestigkeit auf Schmelz zeigte für die Applikatoren mit Platin eine um 10-20 % verbesserte Scherhaftfestigkeit im Vergleich zu den Werten, die mit Applikatoren ohne Additive erzielt werden. Durch die erfindungsgemäßen Applikatoren kann die Haftung der dentalen Materialien in einer für den Anwender unveränderten Anwendung weiter verbessert werden.

Der positive Effekt des Additivs in Bezug auf die Verbesserung der Scherbindungsfestigkeit ist konzentrationsabhängig wie es für Katalysatorsysteme nicht unüblich ist. Da iBOND® Self Etch bei Scherhaftungstests auf Dentin bereits mit handelsüblichen Applikatoren überwiegend kohäsive Brüche in der Zahnhartsubstanz verursacht, kann eine weitere Haftungsverbesserung durch die mit Additiv versehenen Applikatoren mit dieser Methode methodisch nicht nachgewiesen werden. Die Kontrollwerte für iBOND® Self Etch auf Dentin liegen bereits an der oberen Grenze des Bereichs für den sich die Scherhaftung als Methode eignet.

Entsprechend einer bevorzugten Ausführungsform der Erfindung weist mindestens ein Additiv eine Partikelgröße kleiner gleich 100 nm auf, insbesondere größer gleich 1 nm. Alternativ oder zusätzlich liegt mindestens ein Additiv nanopartikulär vor, insbesondere mit einer Partikelgröße kleiner gleich 500 nm bis größer gleich 1 nm, bevorzugt kleiner gleich 250 bis 5 nm, besonders bevorzugt kleiner gleich 100 bis 5 nm. Vorteilhaft ist, dass das mindestens eine Additive mindestens ein nanopartikuläres Metall und/oder mindestens eine Metall enthaltende Verbindung ist, vorzugsweise umfasst ein Additiv eine alkalische Komponentet, wobei dessen Partikelgröße vorzugsweise kleiner gleich 100 nm ist, wie von 100 bis 1 nm.

Entsprechend einer Ausführungsform kann ein Mikro-Applikator einen Körper mit einem distalen und einem proximalen Ende aufweisen, wobei an dem proximalen Ende ein Applikationsmittel vorgesehen ist und vorzugsweise an dem distalen Ende ein Griffelement oder Griffende vorgesehen ist.

Ein bevorzugter Applikator ist vorzugsweise aus einem Polymer, insbesondere aus einem inerten Polymer, das vorzugsweise inert gegenüber dentalen Materialien, wie selbstätzenden Adhäsiven ist, insbesondere ist der Körper des Applikators aus diesen Polymeren. Bevorzugte Polymere umfassen thermoplastische Polymere, insbesondere umfassend PE, PP, HDPE, LDPE, poröses expandiertes Polypropylen (PEPP) und/oder expandiertes Polypropylen (EPP). Der Applikator ist bevorzugt aus einem flexiblen und vorzugweise inerten Material, das weiter bevorzugt gut mit dentalen Materialien benetzbar ist.

Entsprechend ist es bevorzugt, wenn der Applikator ein Applikationsmittel an dem proximalen Ende des Körpers aufweist, dass mindestens ausgewählt ist aus a) Filament, Bürste, Schwamm, Beflockung, Kamm, Bürste, Knäul, Pinsel, Spatel, Mischpad, textilem Material und/oder poröses Mittel, insbesondere können in dem porösen Mittel die Additive eingelagert oder eingebettet vorliegen, um bei Kontakt mit einer dentalen polymerisierbaren Zusammensetzung freigesetzt zu werden. Vorteilhafte Applikatoren sind aus einem thermoplastischen Polymer und/oder chemisch gegenüber Lösemitteln stabilen Polymer. Bevorzugte Applikationsmittel weisen eine Vielzahl an Filamenten auf. Es kann bevorzugt sein, dass der Applikator einen stabförmigen und massiven Körper aufweist, insbesondere weist der Körper des Applikators keinen inneren Hohlraum auf. Weiter bevorzugt ist der Körper zumindest teilweise zylindrisch und massiv und/oder konisch und massig. Erfindungsgemäß ist der Applikator ein Mikro-Applikator mit einem Körper mit einem distalen und einem proximalen Ende, wobei an dem proximalen Ende ein Applikationsmittel vorgesehen ist und vorzugsweise an dem distalen Ende ein Griffelement oder Griffende vorgesehen ist und der Körper des Applikators stabförmig und massiv ist.

Entsprechend einer besonders bevorzugten Ausführungsform ist das Applikationsmittel des Applikators mit mindestens einem katalytisch aktivem Metall oder mindestens einer katalytisch aktiven metallorganischen Verbindung versehen. Vorzugsweise sind die Metalle ausgewählt aus den Metallen der Platingruppe. Besonders bevorzugt ist das mindestens eine katalytisch aktive Metall oder die mindestens eine katalytisch aktive metallorganische Verbindung ausgewählt aus Eisen, Kobalt, Nickel, Ruthenium, Rhodium, Palladium, Osmium, Iridium, Platin, Silber, Kupfer, Zinn und Zink und die metallorganische Verbindung ausgewählt aus Komplexen der vorgenannten Metalle.

Gleichfalls erfindungsgemäß können die Applikatoren mindestens ein Additiv oder eine Mischung dieser Additive aufweisen ausgewählt aus mindestens einer Metall enthaltenden Verbindung ausgewählt aus Alkalisalzen, Erdalkalisalzen; wie anorganischen Salzen, Alkalimetallhydroxiden, Erdalkalimetallhydroxiden, organischen Alkalisalzen, organischen Erdalkalisalzen, und Metall enthaltenden Puffern. Weitere Beispiele umfassen Ca(OH)₂, Nano-HAP, NaOH. Die basischen Additive ermöglichen eine pH-Wert Erhöhung, vorteilhaft kann der pH-Wert um größer gleich 0,2, 0,5 oder 1,0 angehoben werden, besonders bevorzugt ist eine Neutralisation bei der Anwendung mit sauren dentalen Materialien.

Die nachfolgenden Verbindungen gelten, sofern sie auf Applikatoren abgeschieden sind als ein Hilfsmittel: Carbonsäuren, Salz einer Carbonsäure, Fruchtsäure, Puffer und/oder Base. Diese Verbindungen können einen stabilisierenden Effekt auf die Suspension zur Herstellung der Applikatoren aufweisen und auf die Applikatoren imprägniert werden. In einem selbstätzenden dentalen Material können sie, eingebracht über den Applikator, als Puffer wirken.

Ebenso Gegenstand der Erfindung sind Applikatoren mit mindestens einem Additiv oder einer Mischung dieser Additive umfassend Basen, Basen freisetzende Verbindungen ausgewählt aus Metallen, Metall enthaltenden Verbindungen und metallorganischen Verbindungen umfassend Alkalihydroxid, Erdalkalihydroxid, neutrale Basen, anionische Basen, kationische Basen, Basenbildner, wie CaO, Natriumhydrogencarbonat, Aluminiumhydroxide, oder mit mindestens einem weiteren Additiv umfassend organische Basen und/oder Ammoniak.

Die Puffer können unter anderem ausgewählt sein aus Essigsäure-Acetat-Puffer (pH 3,7 bis 5,7), Phosphatpuffer (pH 5,4 bis 8,0), Ammoniakpuffer (pH 8,2 bis 10,2), Citronensäure- oder Citratpuffer, TRIS: Tris(hydroxymethyl)-aminomethan (pH 7,2 bis 9,0), HEPES: 4-(2-Hydroxyethyl)-1-piperazinethanesulfonsäure (pH 6,8 bis 8,2), HEPPS: 4-(2-Hydroxyethyl)-piperazin-1-propansulfonsäure (pH 7,3 bis 8,7), MES: 2-(N-Morpholino)ethansulfonsäure (pH 5,2 bis 6,7), Barbital-Acetat-Puffer nach Michaelis (pH 2,6 bis 9,2) oder einem Milchsäure-Puffersystem.

Eine als Aktivator oder als weiteres Additiv dienende Verbindung, die auf dem Applikator aufgebracht sein kann, kann die folgenden Verbindungen oder Gemische umfassen: aromatische organische Säure und/oder ein Salz davon, wie beispielsweise eine organische Sulfinsäure und/oder ein Salz davon oder Barbitursäure und/oder ein Derivat davon. Die organische Sulfinsäure oder ein Salz davon ist Sulfinsäure oder ein herkömmliches Alkalimetallsalz, Erdalkalimetallsalz, Aminsalz oder Ammoniumsalz von Sulfinsäure. Als Alkalimetallsalze können ein Lithium-, Natrium-, Kaliumsalz oder dergleichen ausgewählt werden. Als Erdalkalimetallsalz können Magnesium-, Calcium-, Strontium-, oder ein Bariumsalz oder dergleichen ausgewählt werden. Als Aminsalz kommen ein primäres Aminsalz wie beispielsweise Methylamin, Ethylamin, Propylamin, Butylamin, Anilin, Toluidin, Phenylendiamin oder Xylylendiamin in Frage; als ein sekundäres Aminsalz kommen beispielsweise Dimethylamin, Diethylamin, Dipropylamin, Dibutylamin, Piperidin, N-Methylanilin, N-Ethylanilin, Diphenylamin oder N-Methyltoluidin in Betracht; oder ein tertiäres Amin wie beispielsweise Trimethylamin, Triethylamin, Pyridin, N,N-Dimethylanilin, N,N-Di(hydroxyethyl)anilin, N,N-Diethylamin, N,N-Dimethyltoluidin, N,N-Diethyltoluidin oder N,N-(Hydroxyethyl)toluidin. Typische Ammoniumsalze umfassen ein Ammonium-, Tetramethylammonium-, Tetraethylammonium-, Tetrapropylammoniumsalz oder Trimethylbenzylammoniumsalz.

Bevorzugte Beispiele für eine organische Sulfinsäure schließen Alkansulfinsäuren ein, wie beispielsweise Ethansulfinsäure, Propansulfinsäure, Hexansulfinsäure, Octansulfinsäure, Decansulfinsäure und Dodecansulfinsäure; alicyclische Sulfinsäuren wie beispielsweise Cyclohexansulfinsäure.

Der Aktivator kann ausgewählt sein aus den folgenden aromatischen organischen Säuren und/oder einem Salz, im Kit ausgewählt aus einer aromatischen organischen Säure und/oder einem Salz davon, wie beispielsweise eine organische Sulfinsäure und/oder ein Salz davon oder Barbitursäure und/oder ein Derivat davon. Als Salze der Sulfinsäure kommen die vorstehend genannten Alkali- oder Erdalkalisalze, Amin- sowie Ammonium-Salze in Betracht.

Vorteilhafte Sulfinsäuren umfassen als organische Sulfinsäure eine Alkansulfinsäure, wie beispielsweise Ethansulfinsäure, Propansulfinsäure, Hexansulfinsäure, Octansulfinsäure, Decansulfinsäure und Dodecansulfinsäure; alicyclische Sulfinsäuren, wie beispielsweise Cyclohexansulfinsäure und Cyclooctansulfinsäure; sowie aromatische Sulfinsäuren, wie beispielsweise Benzolsulfinsäure, o-Toluolsulfinsäure, p-Toluolsulfinsäure, Ethylbenzolsulfinsäure, Decylbenzolsulfinsäure, Dodecylbenzolsulfinsäure, Chlorbenzolsulfinsäure und Naphthalinsulfinsäure.

Bevorzugte Beispiele für die organischen Sulfinate umfassen Lithiumbenzolsulfinat, Natriumbenzolsulfinat, Kaliumbenzolsulfinat, Magnesiumbenzolsulfinat, Calciumbenzolsulfinat, Strontiumbenzolsulfinat, Bariumbenzolsulfinat, Butylaminbenzolsulfinat, Anilinbenzolsulfinat, Toluidinbenzolsulfinat, Phenylendiaminbenzolsulfinat, Diethylaminbenzolsulfinat, Diphenylaminbenzolsulfinat, Triethylaminbenzolsulfinat, Ammoniumbenzolsulfinat, Tetramethylammoniumbenzolsulfinat und Trimethylbenzylammoniumbenzolsulfinat. Ferner kommen bevorzugt in Betracht Lithium-o-toluolsulfinat, Natrium-o-toluolsulfinat, Kalium-o-toluolsulfinat, Calcium-o-toluolsulfinat, Cyclohexylamin-o-toluolsulfinat, Anilin-o-toluolsulfinat, Ammonium-o-toluolsulfinat, Tetraethylammonium-o-toluolsulfinat, Lithium-p-toluolsulfinat, Natrium-p-toluolsulfinat, Kalium-p-toluolsulfinat, p-Toluolsulfinsäure, Barium-p-toluolsulfinat, Ethylamin-p-toluolsulfinat, Toluidin-p-toluolsulfinat, N-Methylanilin-p-toluolsulfinat, Pyridin-p-toluolsulfinat, Ammonium-p-toluensulfinat, Tetramethylammonium-p-toluolsulfinat, Natrium-naphthalinsulfinat, Strontium-naphthalinsuflinat, Triethylamin-naphthalinsulfinat, N-Methyltoluidin-naphthalinsulfiniat, Ammonium-naphthalinsulfinat, Trimethylbenzylammonium-naphthalinsulfinat und dergleichen, die als organisches Sulfinat verwendet werden. Besonders bevorzugt ist Natrium-Toluolsulfinat.

Gleichfalls kann der mindestens eine Aktivator auch im Kit ausgewählt sein aus einem Barbitursäurederivat. Die Barbitursäurederivate sind vorteilhaft ausgewählt aus 1,3,5-Trimethylbarbitursäure, 1,3,5-Triethylbarbitursäure, 1,3-Dimethyl-5-ethylbarbitursäure, 1,5-Dimethylbarbitursäure, 1-Methyl-5-ethylbarbitursäure, 1-Methyl-5-propylbarbitursäure, 5-Ethylbarbitursäure, 5-Propylbarbitursäure, 5-Butylbarbitursäure, 5-Methyl-1-butylbarbitursäure, 1-Benzyl-5-phenylbarbitursäure, 1-Cyclohexyl-5-ethylbarbitursäure oder einem Alkalimetallsalz davon. Bevorzugte Konzentrationen des Barbitursäurederivats liegen zwischen 0,1-10 Gew.-% in Bezug auf die Gesamtzusammensetzung des dentalen Haftmittels.

Die vorgenannten Aktivatoren wie aromatische organische Säuren oder Barbitursäurederivate werden vorzugsweise alleine unter neutralen oder alkalischen Bedingungen aufgelöst. Die Imprägnierung des Applikators kann mittels Tauchen, Sprühen sowie weiterer dem Fachmann geläufiger Methoden erfolgen. Auch ein Aufsprühen eines klebrigen Pulvers ist möglich. Je nach geplanter Anwendung kann das mindestens eine Additiv mit weiteren Hilfsstoffen aufgetragen werden, die die Freisetzung des mindestens einen Additivs zeitlich beeinflussen. Dies kann eine Retardierung oder eine Beschleunigung sein, bspw. indem Zerfallsmittel, eingesetzt werden.

Weitere ebenfalls geeignete Additive, die zusätzlich oder alternativ auf dem Applikator bereitgestellt werden können, umfassen ein Hilfsmittel, Aktivator und/oder mindestens ein weiteres Additiv, wobei die Hilfsmittel umfassen Carbonsäure, Salz einer Carbonsäure, Fruchtsäure, Puffer, hygroskopische Verbindungen, insbesondere hygroskopische Salze, wie (CaCl₂) und/oder das mindestens eine weitere Additiv ist ausgewählt aus Basen und Basen freisetzenden Verbindungen.

Abhängig von der Stabilität der Additive werden die Applikatoren einzeln oder zusammen luftdicht und/oder lichtgeschützt verpackt. Bevorzugt werden die Applikatoren in einer vor Licht schützenden und/oder luftdichten Folie geblistert etc.

Ebenso Gegenstand der Erfindung ist ein Verfahren zur Herstellung eines dentalen Mikro-Applikators, sowie ein Applikator erhältlich nach diesem Verfahren, insbesondere eines vorstehenden Applikators, indem
(i) ein dentaler Mikro-Applikator mit einem Körper mit einem distalen und einem proximalen Ende mit einem Applikationsmittel am proximalen Ende
(ii) mit dem Applikationsmittel mit mindestens einem Additiv ausgewählt aus (i) Metallen, (ii) Metall enthaltenden Verbindungen und (iii) metallorganischen Verbindungen, wobei die Metall enthaltenden Verbindungen ausgewählt sind aus Alkalisalzen, Erdalkalisalzen, anorganischen Salzen der Alkalimetalle, anorganischen Salzen der Erdalkalimetalle, organischen Alkalisalzen, organischen Erdalkalisalzen, Basenbildnern und Metall enthaltenden Puffern, zur Beeinflussung der Haftung dentaler Materialien in Kontakt gebracht wird.

Vorteilhaft wird das mindestens eine Additiv bereitgestellt in (ii) einer Suspension, Dispersion, Paste, Lösung, Sprühtrocknung oder das mindestens eine Additiv wird pulverförmig bereitgestellt.

Weiter ist es bevorzugt, wenn im Schritt (ii) das Applikationsmittel mit einem nanopartikulären Additiv in Kontakt gebracht wird. Dabei ist es bevorzugt, wenn das Additiv, insbesondere das metallische Additiv, besonders bevorzugt Platin, vorzugsweise mit einer Partikelgröße kleiner gleich 500 nm bis größer gleich 1 nm vorliegt, bevorzugt kleiner gleich 250 bis 5 nm, besonders bevorzugt kleiner gleich 100 bis 5 nm oder von 50 bis 2 nm. Vorteilhaft wird mindestens ein nanopartikuläres Metall und/oder mindestens eine nanopartikuläre Metall enthaltende Verbindung vorgelegt. Vorzugsweise kann eine alkalische Komponente, insbesondere mit einer Partikelgröße kleiner gleich 100 nm eingesetzt werden. Wie vorstehend erläutert wird das mindestens eine Additiv vorzugsweise mit einer Partikelgröße kleiner gleich 100 nm in Kontakt gebracht, die insbesondere in einer Suspension vorliegt. Alternativ können die Additive als Lösung, wie als wässrige Lösung, oder einem Gemisch Lösemittel/Wasser im Verfahren angewendet werden. Besonders bevorzugt wird in dem Verfahren in (ii) das nanopartikuläre Metall in einer Suspension eingesetzt.

Entsprechend einer besonders bevorzugten Verfahrensvariante wird das Metall in einer Suspension mit einem Gehalt in Bezug auf die Gesamtzusammensetzung der Suspension von 1,0 bis 1,0 • 10⁻⁸ Gew.-% eingesetzt, insbesondere wird es von 1,0• 10⁻¹ bis 1,0 • 10⁻⁶ Gew.-%, bevorzugt von 1,0• 10⁻³ bis 1,0 • 10⁻⁵ Gew.-%, besonders bevorzugt von 1,0• 10⁻³ bis 2,5 • 10⁻⁴ Gew.-%, erfindungsgemäß von 1,0• 10⁻³ bis 2,2 • 10⁻³ Gew-%, verwendet.

Erfindungsgemäß wird in dem Verfahren zur Herstellung des Applikators vorzugsweise ein Applikator mit dem (ii) Applikationsmittel mit mindestens einem Additiv umfassend mindestens eine Metall enthaltende Verbindung in Kontakt gebracht, die ausgewählt ist aus Alkalisalzen, Erdalkalisalzen, anorganischen Salzen der Alkalimetalle, anorganischen Salzen der Erdalkalimetalle; insbesondere Alkalimetallhydroxiden, Erdalkalimetallhydroxiden; organischen Alkalisalzen, organischen Erdalkalisalzen, Basenbildnern und Metall enthaltenden Puffern.

Die imprägnierten Applikatoren werden vorzugsweise getrocknet.

Gleichfalls Gegenstand der Erfindung ist ein Kit umfassend einen a) Applikator, wie vorstehend beschrieben oder erhältlich nach einem erfindungsgemäßen Verfahren und b) ein dentales Adhäsiv, insbesondere ein polymerisierbares dentales Adhäsiv. Das Kit umfasst vorzugsweise einen a) Applikator umfassend mindestens ein Additiv ausgewählt aus (i) Metallen, (ii) Metall enthaltenden Verbindungen und (iii) metallorganischen Verbindungen, wobei die Metall enthaltenden Verbindungen ausgewählt sind aus Alkalisalzen, Erdalkalisalzen, anorganischen Salzen der Alkalimetalle, anorganischen Salzen der Erdalkalimetalle, organischen Alkalisalzen, organischen Erdalkalisalzen, Basenbildnern und Metall enthaltenden Puffern, zur Beeinflussung der Haftung dentaler Materialien, insbesondere mindestens Platin, insbesondere Platinschwamm. Vorzugsweise weist der Applikator im Kit weitere Additive wie einen Aktivator, wie eine Sulfinsäure oder ein Salz einer Sulfinsäure und/oder eine Base auf.

Erfindungsgemäße Adhäsive sind vorzugsweise selbstätzende dentale Adhäsive.

Bevorzugt umfasst das dentale Adhäsiv mindestens ein A) radikalisch polymerisierbares Monomer mit einer Säuregruppe im Molekül, B1) optional einen Photosensibilisator, und/oder optional B2) ein Peroxid, C) ein mit Wasser mischbares Lösemittel, wie Alkohol, Keton, Ester, Ketal, Isopropylidenglycerin, Ethanol, vorzugsweise Aceton; und D) Wasser.

Weiter kann das Kit umfassen E) mindestens ein radikalisch polymerisierbares Monomer ohne Säuregruppe, das vorzugsweise in Wasser nicht oder kaum löslich ist (kleiner 2 g/ 100 ml H₂O).

Die radikalisch polymerisierbaren Monomere mit einer Säuregruppe im Molekül gemäß A), auch saure Komponente genannt, umfassen polymerisierbare Monomere mit mindestens einer, vorzugsweise mehreren Ethylen-Gruppen sowie mindestens eine Carbonsäure-, Carbonsäureanhydridgruppe, Phosphorsäuregruppe und/oder Sulfonsäuregruppe.

Monofunktionelle polymerisierbare Monomere mit einer Carbonsäure- oder Carbonsäureanhydridgruppe im Molekül können ausgewählt sein aus Monocarbonsäuren, Dicarbonsäuren, Tricarbonsäuren, Tetracarbonsäuren, Polycarbonsäuren und Anhydriden davon. Bevorzugte Verbindungen können Carbonsäuren und/oder Anhydride sein wie Maleinsäure, p-Vinylbenzoesäure, 11-(Meth)acryloyloxy-1,1-undecandicarbonsäure (MAC-10), 1,4-Di(meth)acryloyloxyethylpyromellithsäure, 6-(meth)acryloyloxyethylnaphthalen-1,2,6-tricarbonsäure, 4-(Meth)acryloyloxymethyltrimellithsäure und Anhydride davon, 4-(Meth) acryloyloxyethyltrimellithsäure und dessen Anhydrid, 4-(Meth)acryloyloxybutyltrimellithsäure und dessen Anhydrid, 4-[2-Hydroxy-3-(meth)acryloyloxy]butyltrimellithsäure und dessen Anhydrid, 2,3-Bis(3,4-dicarboxybenzoyloxy)propyl(meth)acrylat, 2-, 3-, oder 4-(Meth)acryloyloxybenzoesäure, N-O-Di(meth)acryloyloxytyrosin, O-(Meth)acryloyloxytyrosin, N-(Meth)acryloyloxytyrosin, N-(Meth)acryloyloxyphenylalanin, N-(Meth)acryloyl-p-amin-benzoesäure, N-(Meth)acryloyl-O-aminbenzoesäure, Addukt aus Glycidyl(meth)acrylat mit N-Phenylglycin oder N-tolylglycin, 4-[(2-Hydroxy-3-(meth)acryloyloxypropyl) amino] phthalsäure, 3- oder 4-[N-Methyl-N-(2-hydroxy-3-(meth)acryloyloxypropyl)amino]phthalsäure, (meth)acryloylaminosalicylsäure und (Meth)acryloyloxysalicylsäure. Bevorzugt sind 11-(Meth)acryloyloxy-1,1-undecandicarbonsäure (MAC-10) und 4-Methacryloyloxyethyltrimellithsäure (4-MET) oder deren Anhydrid (4-META). Polyfunktionelle polymerisierbare Monomere mit mindestens zwei Carboxylgruppen im Molekül, die als Komponente (A) verwendbar sind, können umfassen Dicarbonsäuren, Tricarbonsäuren, Tetracarbonsäuren und Derivate davon, wie beispielsweise ein Additionsprodukt aus 2-Hydroxyethyl-(meth)acrylat und Pyromellithdianhydrid (PMDM), ein Additionsreaktionsprodukt aus 2 Mol Hydroxyethyl-(meth)acrylat und 1 Mol Maleinsäureanhydrid oder 3,3',4,4'-Benzophenontetracarbonsäuredianhydrid (BTDA) oder 3,3',4,4'-Biphenyltetracarbonsäuredianhydrid, und 2-(3,4-Dicarboxybenzoyloxy)1,3-di(meth)acryloyloxypropan.

Polymerisierbare Monomere mit mindestens einer Phosphorsäuregruppe im Molekül umfassen beispielsweise 2-(Meth)acryloyloxyethylsäurephosphat, 2- und 3-(Meth)acryloyloxypropylsäurephosphat, 4-(Meth)acryloyloxybutylsäurephosphat, 6-(Meth)-acryloyloxyhexylsäurephosphat, 8-(Meth)acryloyloxyoctylsäurephosphat, 10-(Meth)-acryloyloxydecylsäurephosphat, 12-(Meth)acryloyloxydodecylsäurephosphat, Bis({2-(meth)-acryloyloxyethyl}säurephosphat, Bis {2 oder 3-(meth)acryloyloxypropyl}-säurephosphat, 2-(Meth)acryloyloxyethylphenylsäurephosphat, 2-(Meth)acryloyloxyethyl-p-methoxyphenylsäurephosphat und dergleichen. Die Phosphorsäure in diesen Verbindungen kann durch eine Thiophosphorsäuregruppe ersetzt sein.

Von den vorgenannten Monomeren sind die folgenden bevorzugt 2-(Meth)acryloyloxyethylphenylsäurephosphat und 10-(Meth)acryloyloxydecylsäurephosphat. Die Monomere mit einer Phosphorsäuregruppe können einzeln oder in einer Kombination verwendet werden.

Polymerisierbare Monomere mit einer Sulfonsäuregruppe im Molekül können umfassen 2-Sulfoethyl(meth)acrylat, 2- oder 1-Sulfo-1- oder -2-Propyl(meth)acrylat, 1- oder 3-Sulfo-2-butyl-(meth)acrylat, 3-Brom-2-sulfo-2-propyl(meth)acrylat, 3-Methoxy-1-sulfo-2-propyl(meth)-acrylat, 1,1-Dimethyl-2-sulfoethyl(meth)acrylamid und 2-Methyl-2-(meth)acrylamidpropansulfonsäure, bevorzugt ist 2-Methyl-2-(meth)acrylamidpropsansulfonsäure.

Das polymerisierbare Monomer A) kann eine Säure-Gruppe umfassen die als Salz, wie beispielsweise als monovalentes oder polyvalentes Metallsalz oder Ammoniumsalz vorliegt. Dabei ist es jedoch bevorzugt, wenn das Monomer A) als Säure wirkt, wenn sie in Verbindung mit einer anderen sauren Verbindung verwendet und mit der anderen sauren Verbindung kontaktiert wird. Die obigen Komponenten (A) können einzeln oder in Kombination miteinander verwendet werden.

Bevorzugte Komponenten B1) und/oder B2) umfassen als B1) optional einen Photosensibilisator, und/oder optional B2) ein Peroxid.

Bevorzugte Komponenten (B1) umfassen eine alpha-Ketocarbonylverbindung oder Acylphosphinoxidverbindung. Konkret bevorzugt sind alpha-Diketon, alpha-Ketoaldehyd, alpha-Ketocarboxylsäure, alpha-Ketocarboxylat. Konkret bevorzugt sind alpha-Diketone, wie beispielsweise Diacetyl, 2,3-Pentadion, 2,3-Hexadion, Benzyl, 4,4'-Dimethoxybenzyl, 4,4'-Diethoxybenzyl, 4,4'-Oxybenzyl, 4,4'-Dichlorbenzyl, 4-Nitrobenzyl, alpha-Naphthyl, alpha-Naphthyl, Campherchinon, Campherchinonsulfonsäure, Campherchinoncarbonsäure und 1,2-Cyclohexandion; alpha-Ketoaldehyde, wie beispielsweise Methylglyoxal und Phenylglyoxal; und andere, wie beispielsweise Pyruvinsäure, Benzoylameisensäure, Phenylpyruvinsäure, Methylpyruvat, Ethylbenzoylformiat, Methylphenylpyravat und Butylphenylpyruvicat. Besonders bevorzugt sind alpha-Diketone aufgrund ihrer Stabilität, sowie Diacetyl, Benzyl und Campherchinon.

Des Weiteren umfasst die Komponente B1) Benzoyldimethoxyphosphinoxid, Benzoylethoxyphenylphosphinoxid, 2-Methylbenzoyldiphenylphosphinoxid, 2,4,6-Trimethylbenzoyldiphenylphosphinoxid sowie deren Derivate. Alle photoaktiven Verbindungen können einzeln oder im Gemisch verwendet werden.

Als Peroxid B2) können eingesetzt werden Verbindungen wie Diacetyldiperoxid, Dipropylperoxid, Dibutylperoxid, Dicaprylperoxid, Dilaurylperoxid, Benzoylperoxid (BPO), p,p'-Dichlorbenzoylperoxid, p,p'-Dimethoxybenzoylperoxid, p,p'-Dimethylbenzoylperoxid und p,p'-Dinitrodibenzoylperoxid; und anorganische Peroxide wie beispielsweise Ammoniumpersulfat, Kaliumpersulfat, Kaliumchlorat, Kaliumbromat und Kaliumperphosphat. Bevorzugt ist BPO.

Als Komponente C) ein mit Wasser mischbares Lösemittel können vorzugsweise verwendet werden Alkohol, Keton, Ester, Ketal, Isopropylidenglycerin, wie Ethanol, aber vorzugsweise Aceton.

Die erfindungsgemäße Komponente (C) ist ein wasserlösliches organisches Lösungsmittel. Dieses dient zur gleichförmigen Auflösung oder Dispergierung jeder der obigen Komponenten, das Lösemittel soll inert gegenüber den Komponeten und vorteilhaft flüchtig sein. Es können auch höhere Alkohole verwendet werden wie beispielsweise Ethylenglycol, Propylenglycol und Glycerin.

Die Komponente E) umfasst vorzugsweise mindestens ein radikalisch polymerisierbares Monomer ohne Säuregruppe, das insbesondere in Wasser nicht oder kaum löslich ist. Bevorzugte radikalisch polymerisierbare Monomere ohne Säuregruppe, die von der Komponente (A) verschieden sind umfassen aromatische Vinylverbindungen, wie beispielsweise Styrol und Divinylbenzol; Vinylester wie beispielsweise Vinylacetat; aliphatische Ester von (Meth)acrylsäure, wie beispielsweise Methyl(meth)acrylat, Ethyl(meth)acrylat, Propyl(meth)acrylat, Butyl(meth)acrylat, Pentyl(meth)acrylat, Hexyl(meth)acrylat, Neopentylglycoldi(meth)acrylat und Trimethylolpropan-tri(meth)acrylat; aromatische Ester wie beispielsweise Phenyl(meth)acrylat; aromatische (Meth)acrylate wie beispielsweise 2-Hydroxy-3-phenoxypropyl(meth)acrylat, ein Addukt aus 1 Mol Bisphenol A mit 2 Mol Glycidyl(meth)acrylat (Bis-GMA), ein Kondensat aus 1 Mol eines Additionspolymers aus Bisphenol A mit Glycidylether und 2 Mol (Meth)acrylsäure und ein Kondensat aus 1 Mol eines Addukts aus Bisphenol A mit Ethylenoxid und 2 Mol (Meth)acrylsäure (Anzahl der Additionsketten von Ethylenoxid m + n = 2,6); urethanbindungshaltige Methacrylate, wie beispielsweise 2-(Meth)acryloyloxy-ethylisocyanat und ein Addukt (UDMA) aus 2 Mol Hydroxy-ethyl(meth)acrylat mit 1 Mol 2,2,4-(oder 2,4,4-) trimethyl-1,6-hexamethylen-diisocyanat; aliphatische (Meth)acrylsäureester wie beispielsweise 1,6-Hexamethylen-dimethacrylat (1,6-HX), Neopentylglycoldi(meth)acrylat und Trimethylolpropan-tri(meth)acrylat; Polyethylen-glycol-di(meth)acrylate (Kettenlänge n = weniger als 6) wie beispielsweise Ethylenglycol-di(meth)acrylat, Diethylenglycoldi(meth)acrylat und Triethylenglycoldi(meth)acrylat; und Polypropylenglycoldi(meth)acrylate (Kettenzahl n = 12 oder weniger) wie beispielsweise Propylenglycol-di(meth)acrylat, Dipropylenglycoldi-(meth)acrylat, Tripropylenglycoldi (meth)acrylat und Nanopropylenglycoldi(meth)acrylat. Erfindungsgemäße Komponenten E) umfassen Monomere, die mindestens eine (Meth-)acrylat-Gruppe umfassen ausgewählt aus Methylmethacrylat, Ethylemethacrylat, Propylmethacrylat, Butylmethacrylat, n-Hexylmethacrylat, 2-Phenoxyethylmethacrylat, Isobornylmethacrylat, Isodecylmethacrylat, Polypropylen-glykol-mono-methacrylat, Tetrahydrofuryl-methacrylat, Polypropylen-glykol-mono-methacrylat, Methylacrylat, Ethylenacrylat, Propylacrylat, Butylacrylat, n-Hexylacrylat, 2-Phenoxyethylacrylat, Isobornylacrylat, Isodecylacrylat, Polypropylen-glykol-mono-acrylat, Tetrahydrofuryl-acrylat, Polypropylen-glykol-mono-acrylat, Hydroxyethylacrylat, Hydroxy-propylacrylat, Hydroxyethylmethacrylat, Hydroxypropylmethacrylat, oder Mischungen davon oder mindestens einen Vernetzer umfassen. Typische Vernetzer sind BDMA, 1,4-Butandiol-dimethacrylat (1,4-BDMA) oder Pentaerythritol-tetraacrylat, Urethandimethacrylat (UDMA), Bis-GMA-Monomer (Bisphenyl-A-Glycidyl-Methacrylat). Sowie die Verwendung von Verdünnungsmitteln (dünnflüssige Acrylate, wie Triethylenglycoldimethacrylat (TEGDMA) und Diethylenglycoldimethacrylat (DEGMA), usw.

Bevorzugte (Meth-)acrylate mit mindestens zwei (Meth-)acrylat-Gruppen sind ausgewählt aus Ethandioldimethacrylat, Tetraethylenglykoldimethacrylat, Diethylenglykoldimethacrylat, Ethylenglykoldimethacrylat, Polyethylenglykoldimethacrylat (400) oder (600), Butandioldimethacrylat, Hexandioldimethacrylat, Decandioldimethacrylat, Dodecandioldimethacrylat, 1,3-Butylenglykoldimethacrylat, Dipropylglykolmethacrylat, Bisphenol-A-dimethacrylat, Bisphenol-A-dimethacrylat Derivat, wie ethoxyliertes 2-Bisphenol-A-dimethacrylat, Trimethylolpropantrimethacrylat, Triethylenglykoldimethacrylat, 2,2-Bis-4-(3-methacryloxy-2-hydroxy-propoxy)-phenylpropan (Bis-GMA), Tricyclodecandimethanoldimethacrylat, ein Urethanmethacrylat mit mindestens zwei Methacrylat-Gruppen oder einer Mischung enthaltend mindestens eines der (Meth-)acrylate.

Bevorzugte (Meth-)acrylate mit drei bis sechs (Meth-)acrylat-Gruppen sind ausgewählt aus (i) mit drei (Meth-)acrylat-Gruppen aus ethoxyliertem-(15)-Trimethylolpropan-Triacrylat, ethoxyliertem-5-Pentaerythritoltriacrylat, propoxyliertem-(5.5)-Glyceryltriacrylat, Trimethylolpropantrimethacrylat, Tris(2-hydroxyethyl)-isocyanurat-triacrylat, und/oder (ii) mit vier (Meth-) acrylat-Gruppen aus Di-Trimethylolpropan-tetra-acrylat, ethoxyliertem-(4)-Pentaerythritol-tetra-acrylat, Pentaerythritol-tetra-acrylat, Di-Trimethylolpropan-tetra-methacrylat, ethoxyliertem-(4)-Pentaerythritol-tetra-methacrylat, Pentaerythritol-tetra-methacrylat und/oder (iii) mit fünf (Meth-)acrylat-Gruppen aus Di-Pentaerythritol-pentaacrylat, i-Pentaerythritol-pentamethacrylat, Dipentaerythritol pentaacrylate, Di(tetramethylolmethan)-pentamethacrylat und/oder (iv) mit sechs (Meth-)acrylat-Gruppen ein Dipentaerythritolhexa(meth)acrylat. Ebenfalls geeignet sind Oligomere von (Meth-)acrylaten, insbesondere Urethan-di-Acrylat-Oligomer.

Gegenstand der Erfindung ist auch ein Kit umfassend einen Applikator der mindestens ein weiteres Additiv aufweist, das ausgewählt ist aus Metall enthaltenden Verbindungen und metallorganischen Verbindungen, insbesondere Alkalihydroxid, Erdalkalihydroxid, Aluminiumhydroxid, Alkali- oder Erdalkalisalze organischer Verbindungen.

Ebenso Gegenstand der Erfindung ist die Verwendung mindestens eines Additivs ausgewählt aus Metallen, Metall enthaltenden Verbindungen und metallorganischen Verbindungen zur Beeinflussung der Haftung dentaler Materialien, wobei das Additiv an oder auf einem Applikationsmittel vorgesehen ist, indem das mindestens eine Additiv mit einem polymerisierbaren dentalen Adhäsiv in Kontakt gebracht wird. Bevorzugt ist mit dem Applikationsmittel das polymerisierbare dentale Adhäsiv auf eine Dentalfläche aufbringbar. Ferner ist Gegenstand der Erfindung die nicht chirurgische und nicht therapeutische Verwendung mindestens eines Additivs ausgewählt aus (i) Metallen, (ii) Metall enthaltenden Verbindungen und (iii) metallorganischen Verbindungen, wobei die Metall enthaltenden Verbindungen ausgewählt sind aus Alkalisalzen, Erdalkalisalzen, anorganischen Salzen der Alkalimetalle, anorganischen Salzen der Erdalkalimetalle, organischen Alkalisalzen, organischen Erdalkalisalzen, Basenbildnern und Metall enthaltenden Puffern, zur Beeinflussung der Polymerisation, insbesondere der Polymerisation dentaler Materialien, wie dentaler Adhäsive, umfassend mindestens ein polymerisierbares Monomer mit einer Säuregruppe im Molekül, vorzugsweise der radikalischen Polymerisation dentaler Materialien, insbesondere enthaltend Monomere mit einer Säuregruppe, wobei das Additiv an oder auf einem Applikationsmittel vorgesehen ist, indem das mindestens eine Additiv mit einem polymerisierbaren dentalen Adhäsiv in Kontakt gebracht wird.

Die radikalisch polymerisierbaren Monomere mit einer Säuregruppe umfassen (Meth)acrylat(e) und/oder olefinische Urethan basierte dentale Monomere.

Nachfolgend wird die Erfindung anhand von Beispielen näher erläutert, ohne sie auf die Beispiele zu beschränken:

### Beispiel 1: Nanoplatin Mikro-Applikator (hier verwendet: Microbrushes®)

Imprägnierung der Microbrush: Die Mikro-Applikatoren (Microbrush; handelsüblich, unbehandelt) werden mit der beflockten Seite in die Imprägnierlösung (Nano-Platin Suspension mit 0.002% Platin-Gehalt) für 24h gehängt. Zur gleichmäßigen Verteilung der Pt Partikel wird die Suspension während der Imprägnierung gerührt.

Anschließend werden die imprägnierten Mikro-Applikatoren im Wärmeschrank bei 37°C für 24h getrocknet. Die Imprägnierung lässt sich durch eine Wägung mittels Analysenwaage nicht quantifizieren. Die Imprägnierung der Mikro-Applikatoren ist jedoch an einer deutlichen Graufärbung erkennbar.

Prüfung der Wirksamkeit der imprägnierten Mikro-Applikatoren:
Prüfung A) Haftversuche zwischen Zahnschmelz und Komposit (Shear Bond Strength, SBS) Zahnsubstrat: Schmelz; Menschenzahn
Adhäsiv: Heraeus Kulzer GmbH iBOND® Self Etch
Komposit: Heraeus Dental Venus® Diamond A2

Microbrush zum Auftragen des Adhäsivs:
1. Unbehandelt als Referenz
2. Imprägniert mit Nanoplatinlösung

Messverfahren - SBS Prüfung am Schmelz: Das Adhäsiv wurde nach Gebrauchsanweisung am aufgeschliffenen Schmelz verarbeitet. Anschließend wurde das Komposit mittels einer Aufnahmevorrichtung (Ultradentvorrichtung, siehe auch EN ISO 29022) so aufgebracht und polymerisiert, dass ein Kompositzylinder mit einem Durchmesser von 2,38 mm entsteht. Nach einer Lagerung von 24h bei 37°C in Wasser, wurden die Probekörper mittels einer Prüfspannvorrichtung (siehe EN ISO 29022) in der Universalprüfmaschine Zwick Z010 fixiert. Die Steuerung der Prüfmaschine erfolgte mit einem PC unter Verwendung der Zwick Software testXpert. Die Prüfung erfolgte bei einer Vorschubgeschwindigkeit von 1,0mm +/-0,1 bis zum Bruch des Verbundes zwischen Zahn und Kompositzylinder.

Die während des Prüfvorgangs auf die Messdose übertragene Kraft wurde durch den PC aufgenommen und durch die Software als Scherfestigkeit in MPa registriert.

Auswertung der Daten und Ergebnisse der Haftversuche im Überblick: Es wurden 2 Messserien iBOND® Self Etch mit der Nanoplatinmicrobrush verarbeitet und mit der Referenz iBOND® Self Etch mit unbehandeltem Mikro-Applikator verglichen. Bei den Haftversuchen wurden Mittelwerte von 29,3 MPa für die Referenz und 35,9 MPa bzw. 36,0 MPa für die Nanoplatin- Mikro-Applikator (Microbrush)Versuche erhalten.

**SBS [MPa] iBOND® Self Etch auf Schmelz**

| | Referenz | Messserien mit Nanoplatin Mikro-Applikatoren | |
|---|---|---|---|
| | unbehandelte Mikro-Applikatoren | Versuch 1 | Versuch 2 |
| Mittelwert | 29,3 | 35,9 | 36,0 |
| Standardabweichung | 1,8 | 7,6 | 1,8 |

Durch die Anwendung der mit Nanoplatin imprägnierten Microbrush (Mikro-Applikator) ist eine signifikant höhere Haftung von im Mittel bis zu 23% am Schmelz erreichbar.

Prüfung B: Polymerisationsumsatz bzw. Doppelbindungsumsatz nach Belichtung
Adhäsiv: HeraeusDental iBOND® Self Etch
Komposit: HeraeusDental Venus A2
Lichtgerät: HeraeusTranslux Powerblue

Microbrush zum Auftragen des Adhäsivs:
1. Unbehandelt als Referenz
2. Imprägniert mit Nanoplatinlösung

### Messverfahren Konversionsrate der Doppelbindungen:

Die Konversionsrate (Prozentsatz der umgesetzten Doppelbindungen) wurde mit einem FTIR-ATR Diamant Spektrometer (Spectrum One, Universal ATR Sample Accessory, Perkin Eimer) bestimmt.

Die FTIR Spektren wurden sofort und 10 Minuten nach der Polymerisation des Adhäsives und der Füllung einer virtuellen Kavität (Füllung überdeckt den ATR Kristall, h = 1,6mm) mit dem Komposit Venus aufgenommen. Die Konversionsrate wurde aus dem Verhältnis der Absorptionsbanden der unpolymerisierten aliphatischen Kohlenstoffdoppelbindung (Wellenlänge = 1638 cm⁻¹) zur polymerisierten Kohlenstoffdoppelbindung berechnet.

Die Spektren wurden derivativ über die 2. Ableitung mit einer Glättung von Punkten, über die totale Höhe des aliphatischen Kohlenstoffdoppelbindungspeaks bei 1638 cm⁻¹ ausgewertet. Das Adhäsiv wurde mit den unbehandelten Microbrush als Referenz und mit dem Nanoplatin-Microbrush auf dem ATR Kristall wie folgt aufgetragen: Zwei Tropfen Bonding wurden in einer Anmischschale vorgelegt. Das Adhäsiv wurde mit dem Microbrush 10 Sekunden gerührt, aufgenommen und in zwei Portionen auf den ATR Kristall aufgetragen. Das Lösemittel wurde 15-20 Sekunden abgedampft und dann für 20 Sekunden mit einem Luftbläser (5bar) so verblasen, dass sich ein starrer Film bildet.

Der unpolymerisierte Adhäsivfilm wird mit einer schwarzen Haube als Lichtschutz abgedeckt und das unpolymerisierte Spektrum aufgenommen. Eine Schablone h = 1,6 mm wurde aufgesetzt, der Adhäsivfilm über dem ATR Kristall wurde für 20 Sekunden polymerisiert. Daraufhin wurde der Adhäsivfilm mit dem Komposit Venus A2 überschichtet, das Komposit mit Folie (die die aktivierte Strahlung durchlässt) abgedeckt und 20 Sekunden polymerisiert. Es erfolgt unmittelbar die Aufnahme des polymerisierten Spektrums <Sofort nach der Belichtung> und das 10 Minuten Spektrum, entsprechend später.

Ergebnisse der Konversionsratenbestimmung Adhäsiv im Überblick: Die Messserie iBOND® Self Etch mit Nanoplatin-Microbrush® verarbeitet wird mit der Referenz iBOND® Self Etch mit unbehandeltem Microbrush® verarbeitet verglichen. Es wurden die Konversionsraten sofort nach der Polymerisation und 10 Minuten nach der Polymerisation jeweils als Mittelwert aus 5 Messungen bestimmt.

**Doppelbindungsumsatz % iBOND® Self Etch sofort nach der Belichtung**

| | Mikro-Applikator | Mikro-Applikatoren |
|---|---|---|
| Mittelwert | 58,5 | 69,0 |
| Standardabweichung | 3,1 | 5,2 |

Durch die Anwendung der mit Nanoplatin imprägnierten Mikro-Applikatoren (Microbrush®) ist direkt nach der Belichtung ein deutlich höherer Doppelbindungsumsatz erreichbar.

**Doppelbindungsumsatz % iBOND Self Etch 10 Minuten nach der Belichtung**

| | Referenz unbehandelte Mikro-Applikatoren | Nanoplatin Mikro-Applikatoren |
|---|---|---|
| Mittelwert | 63,8 | 74,9 |
| Standardabweichung | 3,0 | 4,6 |

Durch die Anwendung der mit Nanoplatin imprägnierten Mikro-Applikatoren ist ein signifikant höherer Doppelbindungsumsatz erreichbar.

### Beispiel 2: Basische Mikro-Applikatoren (Microbrushes®)

Der niedrige pH Wert von All-in-One Adhäsiven verhindert teilweise bis vollständig die Anbindung von selbsthärtenden Werkstoffen. Deshalb soll das Adhäsiv unter Verwendung von alkalisch imprägnierten MB zeitlich in der Lage sein Schmelz/Dentin zu konditionieren, um dann während der Auftragezeit von 20 Sekunden neutral bzw. weniger sauer gestellt zu werden und die Anbindung von selbsthärtenden Werkstoffen zu ermöglichen.

Es werden mit Ca(OH)₂-Lösung imprägnierte Mikro-Applikatoren (Microbrush) hergestellt und das Adhäsiv iBOND Seif Etch mit diesen Microbrushes angewendet.

Herstellung imprägnierter Mikro-Applikatoren_(20 Microbrush): Es wurden 50 ml 0,15%ige wässrige Calciumhydroxidlösung hergestellt (Calciumhydroxid) in Wasser pa. = leicht gesättigte Lösung) hergestellt und die Mikro-Applikatoren (Microbrush®, Pinsel) mit dem beflockten Teil für 1 h unter Rühren in die Lösung getaucht. Anschließend erfolgte eine Trocknung für 24h im Wärmeschrank bei 37°C, getrocknet wurde hängend. Die Griffe der Mikro-Applikatoren wurden gesäubert.

Die Prüfung der Wirksamkeit erfolgte als Messung des pH-Wertes in Abhängigkeit von der Kontaktzeit mit dem Adhäsiv. Dazu wurde der pH-Wert des Adhäsivs vor Kontakt mit den imprägnierten Mikro-Applikatoren, nach 1 Sekunde, 10 Sekunden und 20 Sekunden bestimmt.

Als Kontrollversuch wurde der pH-Wert nach 10 und 20 Sekunden Kontaktzeit mit unbehandelten Mikro-Applikatoren (Microbrush®) bestimmt. Gemäß Gebrauchsanweisung wird iBOND® Self Etch mit einem Mikro-Applikator 20s auf die Zahnhartsubstanz einmassiert, so dass diese Zeitfenster die tatsächliche Anwendungszeit repräsentieren.

Geräte/Materialien: WTW Mircoprozessor pH ION Meter; Schott Instruments Electrode blue Line 16 pH SN Al 15010019 (für kleine Volumina) Zentrifugenvial 1,7 ml; Adhäsiv: iBOND Self Etch; Microbrush® mit Ca(OH)₂; Microbrush® unbehandelt.

Messverfahren: Vorlage von 40 Tropfen Adhäsiv. Messung des pH Wertes: Nacheinander 10 MB (Mikro-Applikator; Microbrush) für 1 Sekunde eintauchen (MB's aufbewahren) pH-Wert für die Kontaktzeit 1 Sekunde messen. Die gleichen MB's werden für weitere 9s in das Adhäsiv unter Rühren eingetaucht (MB's aufbewahren). Der pH-Wert wird für die Kontaktzeit 10s gemessen. Die gleichen MB's werden für weitere 10 Sekunden in das Adhäsiv unter Rühren eingetaucht. Der pH-Wert für die Kontaktzeit 20 Sekunden wird gemessen.

| | Ca(OH)₂-MB | Referenz unbehandelte MB |
|---|---|---|
| Kontaktzeit | pH-Wert | pH-Wert |
| Ohne | 1,55 | 1,55 |
| 1 Sekunde | 1,64 | --- |
| 10 Sekunden | 1,92 | 1,54 |
| 20 Sekunden | 2,00 | 1,56 |

Durch die Anwendung der mit Calciumhydroxid imprägnierten Mikro-Applikatoren ist ein retardierender Effekt auf den pH-Wert erreichbar. Im Zeitfenster der Adhäsivanwendung kann der pH-Wert signifikant erhöht werden.

## Patentansprüche

1. Dentaler Mikro-Applikator mit einem Körper mit einem distalen und einem proximalen Ende mit einem Applikationsmittel am proximalen Ende, **dadurch gekennzeichnet, dass** das Applikationsmittel zumindest teilweise mindestens ein nanopartikuläres Additiv ausgewählt aus
(i) Metallen,
(ii) Metall enthaltenden Verbindungen, wobei die Metall enthaltenden Verbindungen ausgewählt sind aus Alkalisalzen, Erdalkalisalzen, anorganischen Salzen der Alkalimetalle, anorganischen Salzen der Erdalkalimetalle, organischen Alkalisalzen, organischen Erdalkalisalzen, Basenbildnern und Metall enthaltenden Puffern, und
(iii) metallorganischen Verbindungen
zur Beeinflussung der Polymerisation dentaler Materialien aufweist.

2. Applikator nach Anspruch 1, **dadurch gekennzeichnet, dass** das mindestens eine Additiv nanopartikulär mit einer Partikelgröße kleiner gleich 500 nm bis größer gleich 1 nm vorliegt.

3. Applikator nach Anspruch 1 oder 2, **dadurch gekennzeichnet, dass** das Applikationsmittel mit (i) mindestens einem katalytisch aktivem Metall oder (iii) mindestens einer katalytisch aktiven metallorganischen Verbindung versehen ist.

4. Applikator nach einem der Ansprüche 1 bis 3, **dadurch gekennzeichnet, dass** das Applikationsmittel mit (i) mindestens einem katalytisch aktivem Metall oder (iii) mindestens einer katalytisch aktiven metallorganischen Verbindung versehen ist, wobei das Metall ausgewählt ist aus Eisen, Kobalt, Nickel, Ruthenium, Rhodium, Palladium, Osmium, Iridium, Platin, Silber und Kupfer, Zinn und Zink.

5. Applikator nach einem der Ansprüche 1 bis 4, **dadurch gekennzeichnet, dass** das Applikationsmittel zumindest teilweise mit nanopartikulärem Platin imprägniert ist.

6. Applikator nach einem der Ansprüche 1 bis 5, **dadurch gekennzeichnet, dass** das nanopartikuläre Platin eine Partikelgröße kleiner gleich 10 nm aufweist, insbesondere ist das nanopartikuläre Platin Platinschwamm.

7. Applikator nach einem der Ansprüche 1 bis 6, **dadurch gekennzeichnet, dass** der Applikator zusätzlich ein Hilfsmittel, Aktivator und/oder mindestens ein weiteres Additiv aufweist, wobei die Hilfsmittel umfassen Carbonsäure, Salz einer Carbonsäure, Puffer, hygroskopische Verbindungen und/oder das mindestens eine weitere Additiv ausgewählt ist aus Base und Basen freisetzenden Verbindungen.

8. Applikator nach Anspruch 1, **dadurch gekennzeichnet, dass** die Alkalisalze und Erdalkalisalze ausgewählt sind aus Alkalimetallhydroxiden und Erdalkalimetallhydroxiden.

9. Verfahren zur Herstellung eines dentalen Mikro-Applikators nach einem der Ansprüche 1 bis 8, indem
(a) ein dentaler Mikro-Applikator mit einem Körper mit einem distalen und einem proximalen Ende mit einem Applikationsmittel am proximalen Ende
(b) mit dem Applikationsmittel mit mindestens einem nanopartikulären Additiv ausgewählt aus
(i) Metallen,
(ii) Metall enthaltenden Verbindungen und
(iii) metallorganischen Verbindungen,
wobei die Metall enthaltenden Verbindungen ausgewählt sind aus Alkalisalzen, Erdalkalisalzen, anorganischen Salzen der Alkalimetalle, anorganischen Salzen der Erdalkalimetalle, organischen Alkalisalzen, organischen Erdalkalisalzen, Basenbildnern und Metall enthaltenden Puffern,
zur Beeinflussung der Polymerisation dentaler Materialien in Kontakt gebracht wird.

10. Verfahren nach Anspruch 9, **dadurch gekennzeichnet, dass** das nanopartikuläre Additiv mindestens ein Metall ist, ausgewählt aus (i) katalytisch aktiven Metallen und (ii) einer basisch reagierenden Metall-Verbindung.

11. Verfahren nach Anspruch 9 oder 10, **dadurch gekennzeichnet, dass** das Metall Platin ist.

12. Verfahren nach einem der Ansprüche 9 bis 11, **dadurch gekennzeichnet, dass** das Metall in einer Suspension vorliegt und diese zusätzlich ein Hilfsmittel, einen Aktivator und/oder ein weiteres Additiv aufweist, wobei das Hilfsmittel umfasst mindestens eine Carbonsäure, Salz einer Carbonsäure, Puffer und/oder Base.

13. Kit umfassend einen a) Applikator nach einem der Ansprüche 1 bis 8 oder erhältlich nach einem Verfahren nach einem der Ansprüche 9 bis 12 und b) ein dentales Adhäsiv.

14. Kit nach Anspruch 13, **dadurch gekennzeichnet, dass** a) der Applikator als Additiv Platin aufweist.

15. Kit nach Anspruch 13 oder 14, **dadurch gekennzeichnet, dass** das dentale Adhäsiv umfasst A) ein radikalisch polymerisierbares Monomer mit einer Säuregruppe im Molekül, B1) optional einen Photosensibilisator, und/oder optional B2) ein Peroxid, C) ein mit Wasser mischbares Lösemittel und D) Wasser und optional E) mindestens ein radikalisch polymerisierbares Monomer ohne Säuregruppe.

16. Nicht chirurgische und nicht therapeutische Verwendung mindestens eines nanopartikulären Additivs ausgewählt aus
(i) Metallen,
(ii) Metall enthaltenden Verbindungen und
(iii) metallorganischen Verbindungen,
wobei die Metall enthaltenden Verbindungen ausgewählt sind aus Alkalisalzen, Erdalkalisalzen, anorganischen Salzen der Alkalimetalle, anorganischen Salzen der Erdalkalimetalle, organischen Alkalisalzen, organischen Erdalkalisalzen, Basenbildnern und Metall enthaltenden Puffern,
zur Beeinflussung der Polymerisation, insbesondere der radikalischen Polymerisation, dentaler Materialien, wobei das Additiv an oder auf einem Applikationsmittel vorgesehen ist,
indem das mindestens eine Additiv mit einem polymerisierbaren dentalen Adhäsiv in Kontakt gebracht wird, insbesondere ist das das polymerisierbare dentale Adhäsiv mit dem Applikationsmittel auf eine Dentalfläche aufbringbar.

## Claims

1. Dental micro-applicator having a body with a distal and a proximal end with an application aid on the proximal end, **characterised in that** the application aid comprises, at least in part, at least one nano-particulate additive selected from
(i) metals,
(ii) metal-containing compounds, the metal-containing compounds being selected from alkaline salts, alkaline earth salts, inorganic salts of alkali metals, inorganic salts of alkaline earth metals, organic alkaline salts, organic alkaline earth salts, base-forming agents, and metal-containing buffers, and
(iii) organometallic compounds
for affecting the polymerisation of dental materials.

2. Applicator according to claim 1, **characterised in that** the at least one additive is present in nano-particulate form having a particle size of less than or equal to 500 nm to more than or equal to 1 nm.

3. Applicator according to claim 1 or 2, **characterised in that** the application aid is provided with (i) at least one catalytically active metal or (ii) at least one catalytically active organometallic compound.

4. Applicator according to any one of the claims 1 to 3, **characterised in that** the application aid is provided with (i) at least one catalytically active metal or (ii) at least one catalytically active organometallic compound, the metal being selected from iron, cobalt, nickel, ruthenium, rhodium, palladium, osmium, iridium, platinum, silver and copper, tin and zinc.

5. Applicator according to any one of the claims 1 to 4, **characterised in that** the application aid is impregnated, at least in part, with nano-particulate platinum.

6. Applicator according to any one of the claims 1 to 5, **characterised in that** the nano-particulate platinum has a particle size of less than or equal to 10 nm, in particular the nano-particulate platinum is platinum sponge.

7. Applicator according to any one of the claims 1 to 6, **characterised in that** the applicator additionally comprises an excipient, activator and/or at least one further additive, the excipients comprising carboxylic acid, salt of a carboxylic acid, buffer, hygroscopic compounds and/or the at least one further additive being selected from base and bases-releasing compounds.

8. Applicator according to claim 1, **characterised in that** the alkaline salts and alkaline earth salts are selected from alkali metal hydroxides and alkaline earth metal hydroxides.

9. Method for producing a dental micro-applicator according to any one of the claims 1 to 8, in which
(a) a dental micro-applicator having a body with a distal and a proximal end with an application aid on the proximal end
(b) is contacted by means of the application aid with at least one nano-particulate additive selected from
(i) metals,
(ii) metal-containing compounds, and
(iii) organometallic compounds,
the metal-containing compounds being selected from alkaline salts, alkaline earth salts, inorganic salts of alkali metals, inorganic salts of alkaline earth metals, organic alkaline salts, organic alkaline earth salts, base-forming agents, and metal-containing buffers,
for affecting the polymerisation of dental materials.

10. Method according to claim 9, **characterised in that** the nano-particulate additive is at least one metal selected from (i) catalytically active metals and (ii) a metal compound showing an alkaline reaction.

11. Method according to claim 9 or 10, **characterised in that** the metal is platinum.

12. Method according to any one of the claims 9 to 11, **characterised in that** the metal is present in a suspension and said suspension additionally comprises an excipient, an activator and/or a further additive, the excipient comprising at least one carboxylic acid, salt of a carboxylic acid, buffer and/or base.

13. Kit comprising a) an applicator according to any one of the claims 1 to 8 or obtainable according to a method according to any one of the claims 9 to 12, and b) a dental adhesive.

14. Kit according to claim 13, **characterised in that** a) the applicator comprises platinum as additive.

15. Kit according to claim 13 or 14, **characterised in that** the dental adhesive comprises A) a radically polymerisable monomer having an acid group in the molecule, B1) optionally, a photo-sensitiser and/or, optionally, B2) a peroxide, C) a water-miscible solvent, and D) water, and, optionally, E) at least one radically polymerisable monomer without an acid group.

16. Non-surgical and non-therapeutic use of at least one nano-particulate additive selected from
(i) metals,
(ii) metal-containing compounds, and
(iii) organometallic compounds,
the metal-containing compounds being selected from alkaline salts, alkaline earth salts, inorganic salts of alkali metals, inorganic salts of alkaline earth metals, organic alkaline salts, organic alkaline earth salts, base-forming agents, and metal-containing buffers, for affecting the polymerisation, in particular the radical polymerisation, of dental materials, the additive being provided at or on an application aid
by contacting the at least one additive with a polymerisable dental adhesive, in particular the polymerisable dental adhesive is applicable to a dental surface by means of the application aid.

## Revendications

1. Micro-applicateur dentaire ayant un corps avec une extrémité distale et proximale avec un auxiliaire d'application à l'extrémité proximale, **caractérisé en ce que** l'auxiliaire d'application comprend, au moins en partie, au moins un additif nano-particulaire sélectionné à partir
(i) des métaux,
(ii) des composés contenant du métal, les composés contenant du métal étant sélectionnés à partir des sels alcalins, des sels alcalino-terreux, des sels inorganiques des métaux alcalins, des sels inorganiques des métaux alcalino-terreux, des sels alcalins organiques, des sels alcalino-terreux organiques, des agents formant des bases, et des solutions tampon contenant du métal, et
(iii) des composés organométalliques,
pour affecter la polymérisation des matériaux dentaires.

2. Applicateur selon la revendication 1, **caractérisé en ce que** l'au moins additif est présent sous la forme nano-particulaire ayant une taille des particules d'inférieure ou égale à 500 nm à supérieure ou égale à 1 nm.

3. Applicateur selon la revendication 1 ou 2, **caractérisé en ce que** l'auxiliaire d'application est fourni avec (i) au moins un métal ou catalytiquement actif ou (ii) au moins un composé organométallique catalytiquement actif.

4. Applicateur selon l'une des revendications 1 à 3, **caractérisé en ce que** l'auxiliaire d'application est fourni avec (i) au moins un métal catalytiquement actif ou (ii) au moins un composé organométallique catalytiquement actif, le métal étant sélectionné à partir du fer, du cobalt, du nickel, du ruthénium, du rhodium, du palladium, de l'osmium, de l'iridium, du platine, de l'argent et du cuivre, de l'étain et du zinc.

5. Applicateur selon l'une des revendications 1 à 4, **caractérisé en ce que** l'auxiliaire d'application est imprégné, au moins en partie, avec du platine nano-particulaire.

6. Applicateur selon l'une des revendications 1 à 5, **caractérisé en ce que** le platine nano-particulaire a une taille de particules d'inférieure ou égale à 10 nm, en particulière le platine nano-particulaire est une éponge de platine.

7. Applicateur selon l'une des revendications 1 à 6, **caractérisé en ce que** l'applicateur comprend en plus un excipient, un activateur et/ou au moins un autre additif, les excipients comprenant de l'acide carboxylique, du sel d'un acide carboxylique, des solutions tampon, des composés hygroscopiques et/ou l'au moins un autre additif étant sélectionné de la base et des composés libérant des bases.

8. Applicateur selon la revendication 1, **caractérisé en ce que** les sels alcalins et les sels alcalino-terreux sont sélectionnés des hydroxydes de métal alcalin et des hydroxydes de métal alcalino-terreux.

9. Procédé pour produire un micro-applicateur dentaire selon l'une des revendications 1 à 8, dans lequel
(a) un micro-applicateur dentaire ayant un corps avec une extrémité distale et proximale avec un auxiliaire d'application à l'extrémité proximale,
(b) est contacté au moyen de l'auxiliaire d'application avec au moins un additif nano-particulaire sélectionné à partir
(i) des métaux,
(ii) des composés contenant du métal, et
(iii) des composés organométalliques,
les composés contenant du métal étant sélectionnés à partir des sels alcalins, des sels alcalino-terreux, des sels inorganiques des métaux alcalins, des sels inorganiques des métaux alcalino-terreux, des sels alcalins organiques, des sels alcalino-terreux organiques, des agents formant des bases, et des solutions tampon contenant du métal,
pour affecter la polymérisation des matériaux dentaires.

10. Procédé selon la revendication 9, **caractérisé en ce que** l'additif nano-particulaire est au moins un métal sélectionné à partir (i) des métaux catalytiquement actifs et (ii) un composé métallique montrant une réaction alcaline.

11. Procédé selon la revendication 9 ou 10, **caractérisé en ce que** le métal est du platine.

12. Procédé selon l'une des revendications 9 à 11, **caractérisé en ce que** le métal est présent dans une suspension est ladite suspension comprend en plus un excipient, un activateur et/ou un autre additif, l'excipient comprenant au moins un acide carboxylique, du sel d'un acide carboxylique, des solutions tampon et/ou de la base.

13. Kit comprenant a) un applicateur selon l'une des revendications 1 à 8 ou obtenable selon un procédé selon l'une des revendications 9 à 12, et b) un adhésif dentaire.

14. Kit selon la revendication 13, **caractérisé en ce que** a) l'applicateur comprend du platine en tant qu'additif.

15. Kit selon la revendication 13 ou 14, **caractérisé en ce que** l'adhésif dentaire comprend A) un monomère polymérisable radicalairement ayant un groupe d'acide dans la molécule, B1) facultativement, un photo-sensibilisateur et/ou, facultativement, B2) un peroxyde, C) un solvant miscible à l'eau, et D) l'eau, et, facultativement, E) au moins un monomère polymérisable radicalairement sans un groupe d'acide.

16. Utilisation non-thérapeutique et non-chirurgical d'au moins un additive nano-particulaire sélectionné à partir de
(i) des métaux,
(ii) des composés contenant du métal, et
(iii) des composés organométalliques,
les composés contenant du métal étant sélectionnés à partir des sels alcalins, des sels alcalino-terreux, des sels inorganiques des métaux alcalins, des sels inorganiques des métaux alcalino-terreux, des sels alcalins organiques, des sels alcalino-terreux organiques, des agents formant des bases, et des solutions tampon contenant du métal,
pour affecter la polymérisation, en particulière la polymérisation radicalaire, des matériaux dentaires, l'additif étant fourni à ou sur un auxiliaire d'application par contacter l'au moins un additif avec un adhésif polymérisable, en particulière l'adhésif polymérisable est applicable à une surface dentaire au moyen de l'auxiliaire d'application.
